(19) Europäisches Patentamt
European Patent Office
Office européen des brevets

(11) Publication number: 0 117 360
A1

(12) # EUROPEAN PATENT APPLICATION

(21) Application number: 83307752.2

(22) Date of filing: 20.12.83

(51) Int. Cl.³: C 08 L 83/08, A 61 K 7/11, A 61 K 7/06

(30) Priority: 27.12.82 US 453856
26.09.83 US 536042

(43) Date of publication of application: 05.09.84
Bulletin 84/36

(84) Designated Contracting States: DE FR GB

(71) Applicant: DOW CORNING CORPORATION, 3901 S. Saginaw Road, Midland Michigan 48640 (US)

(72) Inventor: Cantrell, Susan Mary, 4512 N. Saginaw Apt. 407, Midland Michigan (US)
Inventor: Homan, Gary Rex, 2801 Dawn Drive, Midland Michigan (US)

(74) Representative: Laredo, Jack Joseph et al, Elkington and Fife High Holborn House 52/54 High Holborn, London, WC1V 6SH (GB)

(54) **Aqueous compositions containing siloxane and a metallorganic additive.**

(57) A composition consisting essentially of (A) a polymer, having at least one nitrogen-hydrogen bond, emulsified in water with at least one surfactant and (B) a water solubilized additive selected from the group consisting of organic zirconates, germanates, titanates, and mixtures thereof is disclosed. This invention further discloses a process for setting and conditioning hair, the improvement comprising applying to the hair the composition as defined above and thereafter drying the hair.

The novelty of this invention lies with the stability of the aqueous siloxane/metallic additive system. This stability allows the composition to be conveniently applied to condition the hair and to hold a set in the hair, but when the hair is washed, the composition reverts to noncrosslinked polymer and acts as a conditioner.

EP 0 117 360 A1

0117360

TITLE MODIFIED
see front page



AQUEOUS SILOXANE/METALLIC ADDITIVE COMPOSITIONS

This invention relates to a composition consisting essentially of (A) a polymer, having at least one nitrogenhydrogen bond, emulsified in water with at least one surfactant and (B) a water solubilized additive selected from the group consisting of organic zirconates, germanates, titanates, and mixtures thereof. This invention further relates to a process for setting and conditioning hair, the improvement comprising applying to the hair the composition as defined above.

The novelty of this invention lies with the stability of the aqueous siloxane/metallic additive system. This stability allows the composition to be conveniently applied to hold a set in the hair, but when the hair is washed, the composition then acts as a conditioner.

A stable aqueous siloxane/metallic additive composition which could serve as a hair set and conditioner would be advantageous over existing systems. In hair care applications, the composition would be applied to wet or dry hair to serve as a conditioner and upon drying, a hair set. Upon washing, though, the composition would be reverted to polymer and act as a wet hair conditioner. An object of this invention is to provide a composition and process capable of performing the above.

A composition described in the article entitled "New Types of Hair Setting Sprays having Semi-Permanent Properties" authored by Fulvio Sardo in Volume 87, American Cosmetics and Perfumery, pages 43-46 (December 1972), discloses the use of alkyltitanates in combination

with silanol endblocked dimethylpolysiloxanes as a hair set. This composition, however, has limited use, at best, as a hair conditioner and setting treatment and is not water stabilized. Furthermore, there is no mention of the composition's capability of reversion to its polymer.

Canadian Patent Number 1,112,181 discloses a hair set composition consisting essentially of aminoalkyl-alkoxysilanes and a titanate ester. This composition also has limited use as a hair conditioner with no mention of reversion capabilities nor water stability.

United States Patent Number 3,832,203 discloses a composition for treating leather comprising an organic solvent solution of (A) a titanate or zirconate, a copolymer of trimethylsiloxane and $SiO_2$ units, a polysiloxane, and (B) an aminoalkyl substituted silane or siloxane. There is no mention of this composition being reversible, parts (A) and (B) cannot be stored together, and it is not stable in water.

British Patent Number 2,058,103 discloses hair grooming agents composed of (alkylamino) methylpolysiloxane and a cationic surfactant with an aqueous carrier. Though the siloxane is somewhat durable, it is not effective in dissipating static charges generated by combing. Furthermore, its hydroxyl endblocking is believed to be the cause of some stability problems and its use is limited to conditioning the hair.

Cationic modified organopolysiloxanes containing quaternary nitrogen is disclosed in Japanese Patent Kokai Sho 55[1980]-66506. These compositions are limited to conditioning and are lacking in providing the hair suppleness, smoothness, softness, and curl retention improvements. Further, the composition is not stable in water.

United States Patent Number 4,388,437 discloses an organopolysiloxane composition, used for treating fibers, consisting essentially of an organopolysiloxane containing amine functionality, a surfactant, a titanate, zirconate, or germanate, an organic acid, and water. The surfactant is needed to emulsify the siloxane. The acid is essential to adjust the pH to a desired range to control the rate of adsorption of the emulsion on the fiber.

An object of this invention is to provide a stable aqueous siloxane/metallic additive composition. A further object of this invention is to provide an improvement to the process of conditioning hair, the improvement comprising applying to hair a composition which imparts conditioning and set holding properties to the hair, as well as a sheen, but when the hair is washed, the composition acts, again, as a conditioner. A further object of this invention is to provide a composition which improves the curl retention properties of hair.

This invention relates to a composition consisting essentially of (A) a polymer, having at least one nitrogenhydrogen bond, emulsified in water with at least one surfactant and (B) a water solubilized additive selected from the group consisting of organic zirconates, germanates, titanates, and mixtures thereof. This invention further relates to a process for setting and conditioning hair, the improvement comprising applying to the hair the composition as defined above.

The novelty of this invention lies with the stability of the aqueous siloxane/metallic additive system. This stability allows the composition to be conveniently applied to condition the hair and at the same time hold a set in the hair, but when the hair is washed,

the composition reverts to noncrosslinked polymer and acts as a conditioner. Setting hair means to curl, wave, control, or straighten hair.

So far as is known at this time, any polymer containing at least one nitrogen-hydrogen bond can be employed for the purposes of this invention. Examples of suitable polymers include polyamides, polyesters, polyethers, polyurethanes, polyalkylenes such as polypropylene, polyethylene and polystyrene, and siloxanes. If the polymer is present in solid form, such as polystyrene, the polymer will have to be dissolved in a compatible solvent, such as toluene or xylene, so the additive can be mixed. Specific examples of suitable polymers include

$$CH_3(C_2H_4)_2\text{-}\overset{H}{N}\text{-}\underset{\overset{\|}{O}}{C}\text{-}O(C_3H_6)_3CH_3 ,$$

$$H_2NCH_2(CH_2COOCH_2)_3CH_3;$$

$$CH_3\text{-}[\underset{CH_3}{\overset{CH_3}{C}} - \underset{OCH_2CH_2NH_2}{CH}]_2CH_3 \quad ;$$

$$H_2NCH_2(CH_2\text{-}\underset{\overset{\|}{O}}{C}\text{-}OCH_2CH_2)_3CH_3; \text{ and}$$

$$CH_3(CH{=}CH)_6\underset{NH_2}{\overset{H}{C}}CH_3 ,$$

which are examples of polyamides, polyesters, polyethers, polyurethanes, and polyalkylenes, respectively. A siloxane polymer is preferred however.

This invention relates more specifically to a composition consisting essentially of (A) an

organosiloxane polymer having an organic substituent containing a nitrogen-hydrogen bond, which polymer is emulsified in water with a surfactant and (B) a water solubilized additive selected from the group consisting of organic titanates, zirconates, germanates, and mixtures thereof. This invention specifically further relates to a process for concurrently setting and conditioning hair comprising (I) coating the hair with a composition comprising (A) an organosiloxane polymer having an organic substituent containing a nitrogen-hydrogen bond, which polymer is emulsified in water with a surfactant and (B) a water solubilized additive selected from the group consisting of organic titanates, zirconates, germanates and mixtures thereof; (II) imposing a desired configuration on the hair; and (III) drying the coated hair in the desired configuration.

Any organosiloxane polymer having an organic substituent containing a nitrogen-hydrogen bond can be employed in the compositions useful in this invention. It is only necessary that the organosiloxane polymer can be emulsified in water with a surfactant. It is generally preferred to employ an organosiloxane polymer containing an organosiloxy unit selected from the group consisting of

$\mathrm{O\overset{R}{\overset{|}{Si}}C_mH_{2m}NR'_2}$, $\mathrm{O_{0.5}\overset{R_2}{\overset{|}{Si}}C_mH_{2m}NR'_2}$,

$\mathrm{O\overset{R}{\overset{|}{Si}}C_mH_{2m}NR'C_nH_{2n}NR'_2}$, $\mathrm{O_{0.5}\overset{R_2}{\overset{|}{Si}}C_mH_{2m}NR'C_nH_{2n}NR'_2}$,

$\mathrm{O_{1.5}SiC_mH_{2m}NR'_2}$, $\mathrm{O_{1.5}SiC_mH_{2m}NR'C_nH_{2n}NR'_2}$,

$O\overset{R}{Si}C_mH_{2m}\overset{O}{\overset{\|}{C}}NR'_2$, $\quad O_{0.5}\overset{R_2}{Si}C_mH_{2m}\overset{O}{\overset{\|}{C}}NR'_2$,

$O\overset{R}{Si}C_mH_{2m}\overset{O}{\overset{\|}{C}}NR'C_nH_{2n}\overset{O}{\overset{\|}{C}}NR'_2$, $O_{0.5}\overset{R_2}{Si}C_mH_{2m}\overset{O}{\overset{\|}{C}}NR'C_nH_{2n}\overset{O}{\overset{\|}{C}}NR'_2$,

$O_{1.5}SiC_mH_{2m}\overset{O}{\overset{\|}{C}}NR'_2$, $\quad O_{1.5}SiC_mH_{2m}\overset{O}{\overset{\|}{C}}NR'C_nH_{2n}\overset{O}{\overset{\|}{C}}NR'_2$,

and mixtures thereof wherein R is selected from the group consisting of monovalent hydrocarbon radicals, substituted monovalent hydrocarbon radicals, hydrogen, and a radical having the general formula -OX wherein X is selected from the group consisting of hydrogen, monovalent hydrocarbon radicals, and substituted monovalent hydrocarbon radicals; R' is selected from the group consisting of hydrogen, monovalent hydrocarbon radicals, and substituted monovalent hydrocarbon radicals; wherein at least one R' per siloxy unit is hydrogen; and m and n have values of 1 to 4.

It should be understood that the divalent radicals represented as $C_mH_{2m}$ and $C_nH_{2n}$ can be either linear or branched hydrocarbon radicals. Specific examples of suitable divalent hydrocarbon radicals represented by $C_mH_{2m}$ and $C_nH_{2n}$ in the above formula include $-(CH_2)_2-$, $-(CH_2)_3-$,

$-(CH_2)-$, $-CH_2CHCH_3CH_2-$ and $\quad -CH_2CH_2\overset{CH_3}{CH}-$.

Specific examples of suitable monovalent hydrocarbon and substituted monovalent hydrocarbon radicals for R, R', and X are alkyl radicals such as the methyl, ethyl, propyl, butyl, amyl, cyclohexyl, decyl, dodecyl, and octadecyl radicals; alkenyl radicals such as the vinyl and

allyl radicals; aryl radicals such as the phenyl and biphenyl radicals; alkaryl and aralkyl radicals such as the tolyl and benzyl radicals; and the corresponding substituted hydrocarbon radicals such as the chloropropyl, 3,3,3-trifluoropropyl, dichlorophenyl, cyanobutyl, and nitrophenyl radicals.

A siloxane polymer having the nitrogen-hydrogen bond in the siloxy unit having the general formula

$$\overset{R}{\underset{|}{}}\hspace{-0.5em}OSiC_mH_{2m}NR'C_nH_{2n}NR'_2$$

wherein R is selected from the group consisting of monovalent hydrocarbon radicals and a radical having the general formula -OX wherein X is selected from the group consisting of hydrogen, monovalent hydrocarbon radicals, and substituted monovalent hydrocarbon radicals; R' is hydrogen, m has a value of 3 or 4 and n has a value of 2 is preferred. If R is a monovalent hydrocarbon radical, it is preferred R be an alkyl radical, with methyl being optimal. If R is the radical having the general formula -OX, it is preferred X be hydrogen. The composition of the other units in the siloxane polymer is not critical for the purpose of this invention.

The other units of the siloxane polymer can be selected from the group consisting of $RSiO_{3/2}$, $R_2SiO$ and $R_3SiO_{1/2}$ units wherein R is as defined above. A siloxane polymer composed primarily of $R_2SiO$ units is preferred, wherein R is the methyl radical. Furthermore the endblocking, composition, and the structure of the polymer is not critical for the purpose of this invention as long as the siloxane polymer contains at least one nitrogen-hydrogen bond. Also, the siloxane polymer containing at least one nitrogen-hydrogen bond can be blended with other

functional siloxanes or non-functional siloxanes, such as dimethylsiloxane or dimethylcyclosiloxane, or their emulsions, to provide further conditioning or less expense.

A siloxane polymer having the general formula

$$R''_3Si(O\underset{R_2}{Si})_a\ (O\overset{R}{\underset{C_mH_{2m}NHC_nH_{2n}NH_2}{Si}})_b\ OSiR''_3$$

wherein R is as defined above, R" is selected from the group consisting of monovalent hydrocarbon radicals, substituted monovalent hydrocarbon radicals, and a radical having the general formula -OX wherein X is selected from the group consisting of hydrogen, monovalent hydrocarbon radicals, and substituted monovalent hydrocarbon radicals; a+b has a value of 1 to 2000 with a having a value of 0 to 1999, and b having a value of 1 to 2000, is preferred. Specific examples of suitable monovalent hydrocarbon radicals and substituted monovalent hydrocarbon radicals for R" are as defined above for R, R',and X. Further preference is given to R and R" being monovalent hydrocarbon radicals, with alkyl being further preferred and methyl being optimal. The value a+b having a value of 50 to 400 with a having a value of 49 to 399 and b having a value of 1 to 30 is also further preferred. Specific examples of suitable siloxane polymers include .

$$\begin{array}{l} \qquad\qquad\quad CH_3 \qquad\quad CH_3 \\ (CH_3)_3Si(OSi)_{96}\ (OSi)_2\ OSi(CH_3)_3 \\ \qquad\qquad\quad CH_3 \qquad\quad CH_2CH(CH_3)CH_2NHCH_2CH_2NH_2, \end{array}$$

$$\begin{array}{l} \qquad\qquad\quad CH_3 \qquad\quad CH_3 \\ (CH_3)_3Si(OSi)_{93.5}(OSi)_{4.5}OSi(CH_3)_3 \\ \qquad\qquad\quad CH_3 \qquad\quad CH_2CH(CH_3)CH_2NHCH_2CH_2NH_2, \end{array}$$

$$\begin{array}{l} \qquad\qquad\quad CH_3 \qquad\quad CH_3 \\ (CH_3)_3Si(OSi)_{90}\ (OSi)_8\ OSi(CH_3)_3 \\ \qquad\qquad\quad CH_3 \qquad\quad CH_2CH(CH_3)CH_2NHCH_2CH_2NH_2, \quad \text{and} \end{array}$$

$$\begin{array}{l} \qquad\qquad\qquad OH \\ HO[SiO]_{98}\ [Si{-}O]_2H \\ \quad (CH_3)_2 \quad (CH_2)_3NH(CH_2)_2NH_2 \quad . \end{array}$$

In order to emulsify the polymer containing at least one nitrogen-hydrogen bond, at least one surfactant must be employed. Surfactants selected from the group consisting of non-ionic surfactants, cationic surfactants, and mixtures thereof are preferred. Examples of suitable non-ionic surfactants include alkyl ethers of polyoxyethylene polymers, alkylaryl ethers of polyoxyethylene polymers, octylphenyl-40-ethyloxylate, trimethylnonylpolyethyleneglycol ether, octylphenyl-10-ethyloxylate, octylphenoxypolyethoxyethanol, 2,6,8-trimethyl-4-nonyloxypolyethyleneoxyethanol, and sorbitan monolaurate. Quaternary ammonium compounds are examples of cationic surfactants, with cetyltrimethylammonium bromide being a specific example. Preference is given to nonionic

surfactants with the surfactant selected from the group consisting of octylphenyl-40-ethyloxylate, octylphenyl-10-ethyloxylate, trimethylnonylpolyethyleneglycol ether, sorbitan monolaurate, octylphenoxypolyethoxyethanol, 2,6,8-trimethyl-4-nonyloxypolyethyleneoxyethanol, and mixtures thereof being optimal.

Preference is given to the following composition for Component (A):

| | | | | | | |
|---|---|---|---|---|---|---|
| 10 to 80 | percent | by | weight | of | the polymer, |
| 19.95 to 89 | " | " | " | " | water, and |
| .05 to 10 | " | " | " | " | the surfactant. |

Component (A) may also contain a neutralizing agent to further stabilize the final composition. It has also been found that the neutralizing agent also provides a more natural, less oily, feeling to the treated hair. Examples of suitable neutralizing agents include mineral and carboxylic acids. Specific examples of suitable mineral acids include hydrochloric, phosphoric, and sulfuric acids. Specific examples of suitable carboxylic acids include acetic acid, succinic acid, citric acid, and 2-ethylhexanoic acid. If the neutralizing agent is a mineral acid, it is preferred it be hydrochloric acid. If the neutralizing agent is a carboxylic acid, it is preferred it be acetic acid. Enough neutralizing agent is added to Component (A) to attain a pH range of 6-8, with 7 being preferred.

The composition also contains a water solubilized additive. "Water solubilized" in this context means that the additive is dissolved in the continuous aqueous phase of the composition. If the additive is readily soluble in water, it may be added to the siloxane emulsion and dissolved directly in the aqueous phase. Alternatively the additive may be dissolved in water and

then added to the siloxane emulsion as an aqueous solution. Furthermore, additives that do not readily dissolve in water can be made water soluble by dissolving them in a mixture of water and an organic cosolvent that is water soluble. This solution of the additive can then be added to the siloxane emulsion to provide the water solubilized additive. An additive solution consisting essentially of 1 to 60 percent by weight of the additive and 40 to 99 percent by weight water is preferred.

The additive solution may also contain 0 to 50 percent by weight of an organic cosolvent that improves the solubility and stability of the additive in the aqueous solution. Suitable cosolvents are selected from the group consisting of water soluble ethers, water soluble organic alcohols, water soluble esters, water soluble organic ketones, and water soluble carboxylic acids. Examples of suitable ethers include methyl ether, ethyl ether, and bis(2-ethoxyethyl)ether. Examples of suitable water soluble alcohols include isopropanol and ethanol. Examples of suitable water soluble esters include propylformate and propylacetate. Examples of suitable water soluble organic ketones include acetone, methylethylketone, and 2-pentanone. Examples of suitable water soluble carboxylic acids include glacial acetic acid, formic acid, and benzoic acid. It is preferred the organic cosolvent be a water soluble organic ketone, with methylethylketone being optimal.

So far as is known at this time, any water solubilized additive selected from the group consisting of organic zirconates, germanates, titanates, and mixtures thereof can be employed for the purpose of this invention. Specific examples of suitable zirconates include tetraethylzirconate, tetradecylzirconate,

tetradodecylzirconate, octyleneglycolzirconate, tetra-2-ethylhexylzirconate, tetraisopropylzirconate, tertiarybutyltrimethylzirconate and mixtures thereof. Specific examples of suitable titanates include tetraethyltitanate, tetradecyltitanate, tetra(octyleneglycolyl)titanate, tetra-2-ethylhexyltitanate, tetradodecyltitanate, tetrabutyltitanate, tetraisopropyltitanate, and mixtures thereof. Specific examples of suitable germanates include tetraethylgermanate, tetraisopropylgermanate, and tetradecylgermanate. Titanates are preferred, however. Titanates selected from the group consisting of alkyl titanates, and titanium chelates are further preferred.

Alkyl titanates have the general formula $Ti(OD)_4$ wherein D is a monovalent hydrocarbon radical or a substituted monovalent hydrocarbon radical.

Titanium chelates have the general formulae

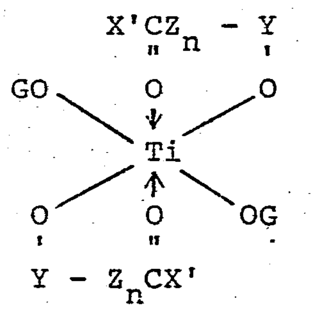

and

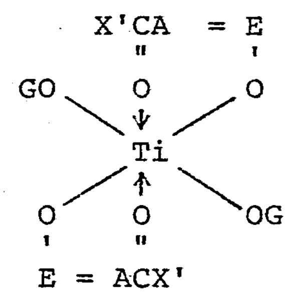

wherein G is selected from the group consisting of hydrogen, monovalent hydrocarbon radicals, and substituted monovalent hydrocarbon radicals, X' is selected from the group consisting of hydrogen, $-OCH_2CH_3$, -OH, the $-O^-NH_4^+$ radical, monovalent hydrocarbon radicals, and substituted monovalent hydrocarbon radicals, Z and Y are divalent hydrocarbon or substituted divalent hydrocarbon radicals, n has the value of 0 to 10, and A and E are trivalent or substituted trivalent hydrocarbon radicals.

Specific examples of suitable monovalent hydrocarbon or substituted monovalent hydrocarbon radicals for D, X', and G are alkyl radicals such as the methyl, ethyl, propyl, butyl, amyl, cyclohexyl, decyl, dodecyl, and octadecyl radicals; alkenyl radicals such as the vinyl and allyl radicals; aryl radicals such as the phenyl and biphenyl radicals; alkaryl and aralkyl radicals such as the tolyl and benzyl radicals; and the corresponding substituted hydrocarbon radicals such as the chloropropyl, 3,3,3-trifluoropropyl, dichlorophenyl, cyanobutyl, and nitrophenyl radicals.

The hydrocarbon and substituted hydrocarbon radicals for Y and Z are divalent radicals. Though the above examples of suitable hydrocarbon and substituted hydrocarbon radicals for D, X' and G are in monovalent form, similar divalent radicals are suitable for Y and Z. Specific examples of suitable divalent hydrocarbon radicals include $-(CH_2)_3-$, $-(CH_2)_4-$, $-(CH_2)_6-$, and $-CH_2CHCH_3CH_2-$.

The hydrocarbon and substituted hydrocarbon radicals for A and E are trivalent radicals. Though the above examples of suitable hydrocarbon and substituted hydrocarbon radicals for D, X', and G are in monovalent form, similar trivalent radicals are suitable for A and E. Specific examples of suitable trivalent hydrocarbon radicals include $=\overset{H}{\overset{|}{C}}-$ and $-\overset{CH_3}{\overset{|}{C}}=$ .

Specific examples of suitable titanates having the general formula $Ti(OD)_4$ include tetraisopropyltitanate, tetra-n-butyltitanate, and tetrakis(2-ethylhexyl)titanate. Specific examples of suitable titanates having the general formula

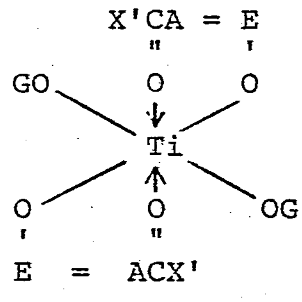

include titanium acetylacetonate and titanium ethyl acetylacetonate. A specific example of a titanate having the general formula

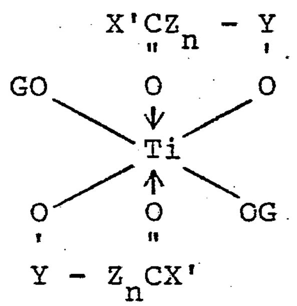

is lactic acid titanium chelate where X' is the $NH_4^+O^-$ radical, n has the value of 0, Y is the $-CHCH_3$ radical and G is hydrogen.

Titanates having the general formula

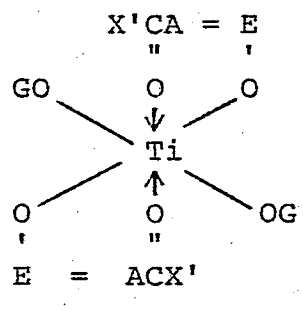

wherein X' and G are monovalent hydrocarbon radicals and A and E are trivalent hydrocarbon radicals are preferred. Further preference is given to X' being a methyl radical, A having the formula

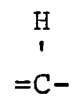 ,

E having the formula

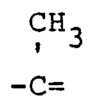

and G having the formula $-C_3H_7$. Titanates having the general formula

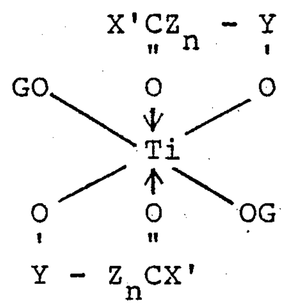

wherein X' is the $NH_4{}^+O^-$ radical, n has the value of 0, Y is the $-CHCH_3$ radical and G is hydrogen, are also preferred.

Generally, it is preferred that the compositions of this invention consist essentially of water: 0.1 to 80 percent by weight of organosiloxane polymer; 0.0005 to 10 percent by weight of the surfactant; 0.005 to 20 percent by weight of the organic titanate, zirconate or germanate; and 0 to 20 percent by weight of organic cosolvent. Even more preferred are compositions consisting essentially of water; 0.5 to 30 percent by weight of organosiloxane polymer; 0.05 to 5 percent by weight of the surfactant; 0.08 to 10 percent by weight of the organic titanate, zirconate or germanate; and 0.1 to 10 percent by weight of organic cosolvent selected from the group consisting of water soluble alcohols, water soluble ketones, water soluble esters, water soluble ethers, water soluble carboxylic acids, and mixtures thereof.

If desired, the composition may also contain 0.01 to 50 percent by weight of an alkoxysilane having at least two alkoxy groups per silicon for added durability. Specific examples of suitable alkoxysilanes include methyltrimethoxysilane and a silane having the formula $NH_2CH_2CH_2NHCH_2CH_2CH_2Si(OCH_3)_3$.

In addition to the essential ingredients, the composition of this invention may include minor quantities of optional materials which are added for specific purposes. Such other ingredients include, but are not limited to, medicaments such as anti-dandruff compounds, solvents, thickening agents, perfumes, sequestering agents, fillers, opacifiers, conditioning agents such as quaternary ammonium compounds, freeze-thaw stabilizers such as ethylene glycol, anti-static agents, and antimicrobial preservatives, of which silicone is one.

The methods of preparation of the components of the present invention are well known to those skilled in the art and detailed elsewhere in the literature. Hence, no time or space need be devoted here to a repetition of such information. As far as is known at this time, the order or method of mixing the prepared components employed is not critical for the purpose of this invention.

Suitable uses for this composition are in the field of hair care and anywhere there is need for a reversible rubber or elastomer. In the field of hair care, preference is given to applying the composition to wet hair. The composition can be applied to the hair in any suitable form such as in thickened form by hand, in viscous form, or by pump spray. The hair may be rinsed with water after the composition is applied to the hair, but it is not necessary. The composition can be applied to the hair either before or after imposing the desired

configuration on the hair. In either case, once the hair is in the desired configuration, it is only necessary that the coated hair be dried to provide a set that holds even when exposed to atmospheric humidity.

The hair can be dried by allowing the volatile components of the composition to evaporate at room temperature. Of course, the hair can also be dried at elevated temperatures if desired. The composition after curing on the hair acts to hold any set in the hair even when exposed to atmospheric humidity, but when the hair is contacted directly with water such as by washing, the set is removed from the hair. Washing hair with a shampoo rather than just water is even more effective in removing the set from the hair. However, sufficient composition remains on the hair even after washing to act as a conditioner for the wet hair. Furthermore, if the hair is reset and dried, the composition again acts to hold the set in the hair although to a somewhat lesser extent than after the initial application of the composition. Although not intending to be bound by this theory, applicants believe that the cured composition when contacted directly with water such as by washing, reverts to the noncrosslinked, uncured polysiloxane which acts as the conditioner for the wet hair. When the hair is again dried however, applicants believe that the composition that remains on the hair again cures to sufficient extent to improve the set holding properties of the hair.

The term "hair" as used in the present invention includes treated and untreated human hair, animal hair, and any type of fiber that needs gloss, ease of combing, set hold, feel of fullness, protection from elements such as sun, and reduced fly-away. Treated hair includes hair that is chemically changed and/or damaged by permanents

and dyes. Setting hair means to curl, wave, control, or straighten hair.

Now in order that those skilled in the art may better understand how the present invention can be practiced, the following examples are given by way of illustration and not by way of limitation. All parts and percents referred to herein are by weight unless otherwise specified.

Example 1

Compositions were prepared for a final 9:1 weight ratio silicone to titanium acetylacetonate. The final composition consisted of 78.26 grams of the polymer emulsion and 21.74 grams of the titanium acetylacetonate solution.

The titanium acetylacetonate solution was prepared by mixing 10 weight percent titanium acetylacetonate, 20 weight percent methylethylketone, and 70 weight percent water.

The polymer emulsion was prepared by emulsifying 25 weight percent of the polymer in 70.1 weight percent water with 4.4 weight percent octylphenyl-40-ethyloxylate and 0.5 weight percent sorbitan monolaurate. The polymers tested were:

(A)

$$(CH_3)_3Si(\underset{CH_3}{\overset{CH_3}{OSi}})_{96}(\underset{CH_2CHCH_3CH_2NHCH_2CH_2NH_2}{\overset{CH_3}{OSi}})_2OSi(CH_3)_3 \;;$$

(B)

$$(CH_3)_3Si(\underset{CH_3}{\overset{CH_3}{OSi}})_{93.5}(\underset{CH_2CHCH_3CH_2NHCH_2CH_2NH_2}{\overset{CH_3}{OSi}})_{4.5}OSi(CH_3)_3 \;;$$

(C)

$$\begin{array}{lll} & CH_3 & CH_3 \\ (CH_3)_3Si & (OSi)_{90} & (OSi)_8OSi(CH_3)_3\ ; \\ & CH_3 & CH_2CHCH_3CH_2NHCH_2CH_2NH_2 \end{array}$$

(D)

$$\begin{array}{lll} & & OH \\ HO & [SiO]_{98} & [SiO]_2H \\ & (CH_3)_2 & (CH_2)_3NH(CH_2)_2NH_2 \end{array}$$

Hair swatches, about 14 grams of Virgin European, natural brown, were then soaked in the solutions two minutes, and then dipped ten times each in two 700 ml distilled water rinses. The treated hair and two water wetted untreated swatches were rolled on 11/16" outer diameter rollers and dried in a 70°C oven for 1¼ hours.

After unrolling the samples, one untreated sample was sprayed with Miss Breck® Ultimate Hold, aerosol hair spray manufactured by American Cyanimid, and shall be denoted as E. The other untreated sample shall be denoted as F.

The curl lengths were then measured and reported as a percent of initial curl length of the untreated sample. The lower the percent curl value, the better the curl retention. The curls were then left exposed to the relative humidity of the environment. The measurements were again taken after 6 hours, and after 3 days. The relative humidity of the environment over the 3 day testing period ranged from 66% to 73% at 23°C.
The results are as follows:

| Sample | Initial | 6 hours | 3 days |
|---|---|---|---|
| A | 86% | 105% | 123% |
| B | 64% | 100% | 127% |
| C | 68% | 105% | 123% |
| D | 82% | 95% | 120% |
| E | 91% | 120% | 127% |
| F | 100% | 136% | 145% |

In conclusion, all treatments had better curl retention than the untreated. As added benefits, the treated hair swatches developed more body, improved wet and dry feel, and improved wet and dry combing ease.

Example 2

Sample A, D and E from Example 1 were washed after the 3 day curl retention test period. Both swatches were dipped ten times and soaked for one minute in 200 grams of 3 weight percent Prell®, manufactured by Proctor and Gamble, Cincinnati, Ohio, Shampoo-in-water solution.

The swatches were then dipped ten times each in two (2) 700 ml distilled water bath. The hair was then rolled on an 11/16" outer diameter rollers and dried in a 70°C oven for 1¼ hours. The silicone treated samples again showed improved curl retention, combing ease and feel, demonstrating the treatment is substantive. Sample E did not retain its curl, nor did it demonstrate any combing ease.

Example 3

The following compositions were prepared to demonstrate the polymers are effective at low concentrations. The titanium acetylacetonate solution and the emulsions were prepared as in Example 1. The polymers tested were

(G)

$$HO[\underset{(CH_3)_2}{SiO}]_{98}[\overset{OH}{\underset{(CH_2)_3NH(CH_2)_2NH_2}{SiO}}]_2-H$$

and

(H)

$$(CH_3)_3Si(\overset{CH_3}{\underset{CH_3}{OSi}})_{96}(\overset{CH_3}{\underset{CH_2CHCH_3CH_2NHCH_2CH_2NH_2}{OSi}})_2OSi(CH_3)_3$$

The final concentrations tested were (1) 3.2 grams of the polymer emulsion of G and H, 0.9 grams of the titanium acetylacetonate solution and 96 grams distilled water and (2) 31.2 grams of the polymer emulsion of H, and 8.8 grams of the titanium acetylacetonate solution, and 60 grams distilled water.

Hair swatches, about 14 grams of Virgin European, natural brown, were then soaked in the solutions two minutes, and then dipped ten times each in two 700 ml distilled water rinses. The treated hair and a waterwetted untreated swatch, Sample I, were rolled on 11/16" outer diameter rollers and dried in a 70°C oven for 1¼ hours.

After unrolling the curl lengths were then measured and reported as a percent of initial curl length of the untreated sample. The lower the percent curl value, the better the curl retention. The measurements were taken initially, after 6 hours, and after 1 day. The relative humidity of the environment was 62% at 23°C. The results are as follows:

| Sample | Initial | 6 hours | 1 days |
|---|---|---|---|
| G | 86% | 105% | 114% |
| H-1 | 95% | 105% | 119% |
| H-2 | 86% | 110% | 119% |
| I | 100% | 121% | 133% |

In conclusion, the treatments were effective in all cases and improvements were observed for combing ease, feel, and body.

Example 4

A zirconium-n-propoxide solution was prepared by mixing 10 percent by weight zirconium-n-propoxide, 20 percent by weight glacial acetic acid, and 70 percent by weight water.

The polymer emulsion was prepared by emulsifying 35 weight percent of

$$(CH_3)_3Si(\underset{CH_3}{\overset{CH_3}{OSi}})_{96}(\underset{CH_2CHCH_3CH_2NHCH_2CH_2NH_2}{\overset{CH_3}{OSi}})_2OSi(CH_3)_3$$

in 58.3 weight percent water with 3.6 weight percent octylphenoxypolyethoxyethanol, 1.7 weight percent 2,6,8-trimethyl-4-nonyloxypolyethyleneoxyethanol, and 1.4 weight percent ethylene glycol.

The composition for testing consisted of 7.8 grams of the zirconium-n-propoxide solution, 28.6 grams of the polymer emulsion, and 63.6 grams of water.

A hair swatch, about 14 grams of Virgin European, natural brown, was then soaked in the composition two minutes, and then dipped ten times each in two 700 ml distilled water rinses. The treated hair was then rolled on a 11/16" outer diameter roller and dried in a 70°C oven for 1½ hours.

The curl length was measured as 93 percent of the initial curl length 6 hours after unrolling the sample. The relative humidity of the environment over the testing period was 68 percent at 75°F.

In conclusion, zirconates in combination with a silicone containing at least one N-H bond do provide curl retention.

Example 5

Compositions were prepared to test the performance of other titanates in polymer emulsions as hair sets.

The titanates tested were:

(A) lactic acid titanium chelate

(B) 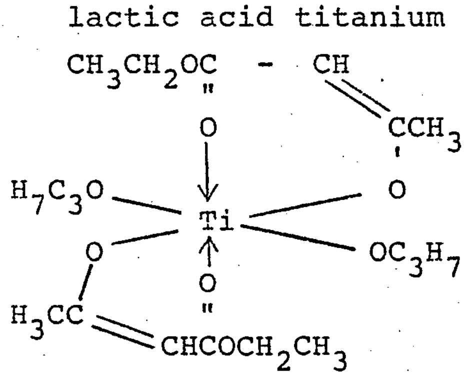

(C) tetraisopropyltitanate

(D) tetra-n-butyltitanate

(E) titanium acetylacetonate

Lactic acid titanium chelate is already water soluble so it was added directly to the polymer emulsion. The other titanates had to be made water soluble by mixing 10 weight percent of the titanate, 20 weight percent of acetic acid, and 70 weight percent water.

The polymer emulsion was prepared by emulsifying 35 weight percent of

$$(CH_3)_3Si(\underset{CH_3}{\overset{CH_3}{\overset{|}{\underset{|}{OSi}}}})_{96}(\underset{CH_2CHCH_3CH_2NHCH_2CH_2NH_2}{\overset{CH_3}{\overset{|}{\underset{|}{OSi}}}})_2OSi(CH_3)_3$$

in 58.3 weight percent water with 3.6 weight percent octylphenyl-40-ethyloxylate, 1.7 weight percent 2,6,8-trimethyl-4-nonyloxypolyethyleneoxyethanol, and 1.4 weight percent ethylene glycol.

The compositions for testing consisted of 28.6 grams of the polymer emulsion, and

(A) 1.4 grams of titanate (A) and 70 grams water;

(B) 10.8 grams of titanate (B) solution and 60.6 grams water;

(C) 6.8 grams of titanate (C) solution and 64.6 grams water;

(D) 13.5 grams of titanate (D) solution and 57.9 grams water; and

(E) 8.1 grams of titanate (E) solution and 63.3 grams water.

Hair swatches, about 14 grams of Virgin European, natural brown, were then soaked in the compositions for two minutes, and then dipped ten times each in two 700 ml distilled water rinses. The treated hairs and two waterwetted untreated swatches were then rolled on 11/16" outer diameter rollers and dried in a 70°C oven for 1¼ hours.

After unrolling the samples the curl lengths were then measured and reported as a percent of initial curl length of the untreated samples. The lower the percent curl value, the better the curl retention. The untreated samples shall be denoted as F and G. Dependent on the sample, the measurements were taken initially,

after 7 hours, and after 2 days, or initially, after 4 hours, and after 45 hours. The relative humidity of the environment during testing ranged from 57% to 73% at 76°F.

| Sample | Initial | 7 Hours | 2 Days |
|---|---|---|---|
| A | 93% | 93% | 91% |
| B | 81% | 85% | 88% |
| E | 96% | 96% | 88% |
| F | 100% | 100% | 100% |

| Sample | Initial | 4 Hours | 45 Hours |
|---|---|---|---|
| C | 86% | 88% | 103% |
| D | 86% | 83% | 90% |
| G | 100% | 100% | 100% |

Example 6

Compositions were prepared to test the performance of compositions where the polymer emulsion was neutralized.

The titanate solution was prepared by mixing 10 weight percent of titanium acetylacetonate, 20 weight percent methylethylketone, and 70 weight percent water.

The polymer emulsion was prepared by emulsifying 35 weight percent of

$$(CH_3)_3Si(\underset{\substack{|\\CH_3}}{\overset{\substack{CH_3\\|}}{OSi}})_{43.5}(\underset{\substack{|\\CH_2CHCH_3CH_2NHCH_2CH_2NH_2}}{\overset{\substack{CH_3\\|}}{OSi}})_{4.5}OSi(CH_3)_3$$

in 60.0 percent water with 4.4 weight percent octylphenyl-40-ethyloxylate and 0.6 weight percent sorbitan monolaurate. The polymer emulsion was then neutralized with

(A) aqueous acetic acid to a pH of 6.8 and

(B) hydrochloric acid to a pH of 6.6.

The final compositions consisted of 28.6 grams of the neutralized polymer emulsion and 11.1 grams of the titanate solution, and 60.3 grams water.

Hair swatches, about 14 grams of Virgin European, natural brown, were then soaked in the solutions for two minutes, and then dipped ten times each in two 700 ml distilled water rinses. The treated hair and one waterwetted untreated swatch sample C, was rolled on 11/16" outer diameter rollers and dried in a 70°C oven for 1¼ hours.

After unrolling the samples, the curl lengths were then measured and reported as a percent improvement over the initial curl length. The higher the percent curl value, the better the curl retention. The measurements were taken after 1½ hours and combing, 4 hours, and 1 day. The relative humidity range of the environment over this testing period was 41 to 51 percent at 76°F.

The results are as follows:

| Sample | 1.5 hours | 4 hrs. | 24 hrs. |
|---|---|---|---|
| A | 26% | 26% | 31% |
| B | 15% | 10% | 11% |
| C | 0 | 0 | 0 |

Claims:

1. A composition consisting essentially of (A) an organosiloxane polymer having an organic substituent containing a nitrogen-hydrogen bond, which polymer is emulsified in water with a surfactant and (B) a water solubilized additive selected from the group consisting of organic titanates, zirconates, germanates and mixtures thereof.

2. A composition as defined in Claim 1 wherein (A) the organosiloxane polymer contains an organosiloxy unit selected from the group consisting of

$O\overset{R}{\overset{|}{Si}}C_mH_{2m}NR'_2$, $O_{0.5}\overset{R_2}{\overset{|}{Si}}C_mH_{2m}NR'_2$,

$O\overset{R}{\overset{|}{Si}}C_mH_{2m}NR'C_nH_{2n}NR'_2$, $O_{0.5}\overset{R_2}{\overset{|}{Si}}C_mH_{2m}NR'C_nH_{2n}NR'_2$,

$O_{1.5}SiC_mH_{2m}NR'_2$, $O_{1.5}SiC_mH_{2m}NR'C_nH_{2n}NR'_2$,

$O\overset{R}{\overset{|}{Si}}C_mH_{2m}\overset{O}{\overset{\|}{C}}NR'_2$, $O_{0.5}\overset{R_2}{\overset{|}{Si}}C_mH_{2m}\overset{O}{\overset{\|}{C}}NR'_2$,

$O\overset{R}{\overset{|}{Si}}C_mH_{2m}\overset{O}{\overset{\|}{C}}NR'C_nH_{2n}\overset{O}{\overset{\|}{C}}NR'_2$, $O_{0.5}\overset{R_2}{\overset{|}{Si}}C_mH_{2m}\overset{O}{\overset{\|}{C}}NR'C_nH_{2n}\overset{O}{\overset{\|}{C}}NR'_2$,

$$O_{1.5}SiC_mH_{2m}\overset{O}{\overset{\|}{C}}NR'_2, \qquad O_{1.5}SiC_mH_{2m}\overset{O}{\overset{\|}{C}}NR'C_nH_{2n}\overset{O}{\overset{\|}{C}}NR'_2,$$

and mixtures thereof wherein R is selected from the group consisting of hydrogen, monovalent hydrocarbon radicals, substituted monovalent hydrocarbon radicals, and a radical having the general formula -OX wherein X is selected from the group consisting of hydrogen, monovalent hydrocarbon radicals, and substituted monovalent hydrocarbon radicals; R' is selected from the group consisting of hydrogen, monovalent hydrocarbon radicals, and substituted monovalent hydrocarbon radicals; wherein at least one R' per organosiloxy unit is hydrogen; and $\underline{m}$ and $\underline{n}$ have values of 1 to 4 and (B) the additive is an organic titanate.

3. A composition as defined in Claim 2 wherein (A) the organosiloxane polymer contains an organosiloxy unit having the general formula

$$O\overset{R}{\overset{|}{Si}}C_mH_{2m}NR'C_nH_{2n}NR'_2$$

wherein R is selected from the group consisting of monovalent hydrocarbon radicals and a radical having the general formula -OX wherein X is selected from the group consisting of hydrogen, monovalent hydrocarbon radicals, and substituted monovalent hydrocarbon radicals; R' is hydrogen, $\underline{m}$ has a value of 3 or 4, and $\underline{n}$ has a value of 2; and (B) the titanate is a titanium chelate selected from the group consisting of

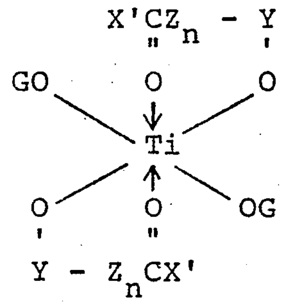

and

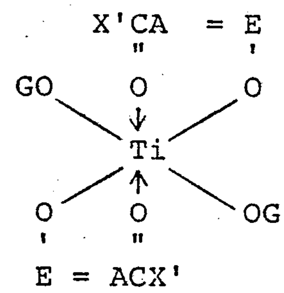

wherein G is selected from the group consisting of hydrogen, monovalent hydrocarbon radicals, and substituted monovalent hydrocarbon radicals, X' is selected from the group consisting of hydrogen, $-OCH_2CH_3$, -OH, the $-O^-NH_4^+$ radical, monovalent hydrocarbon radicals, and substituted monovalent hydrocarbon radicals, Z and Y are divalent hydrocarbon or substituted divalent hydrocarbon radicals, <u>n</u> has the value of 0 to 10, and A and E are trivalent or substituted trivalent hydrocarbon radicals.

4. A composition as defined in Claim 3 wherein (A) the organosiloxane polymer has the general formula

$$R''_3Si(O\underset{R_2}{Si})_a(O\overset{R}{\underset{C_mH_{2m}NHC_nH_{2n}NH_2}{Si}})_bOSiR''_3$$

wherein R is selected from the group consisting of alkyl radicals and a radical having the general formula -OX wherein X is hydrogen; $R''$ is selected from the group consisting of monovalent hydrocarbon radicals, substituted monovalent hydrocarbon radicals, and a radical having the general formula -OX wherein X is selected from the group consisting of hydrogen, monovalent hydrocarbon radicals, and monovalent substituted hydrocarbon radicals; <u>a</u>+<u>b</u> has a value of 1 to 2000 with <u>a</u> having a value of 0 to 1999 and <u>b</u> having a value of 1 to 2000.

5. A process for concurrently setting and conditioning hair comprising

(I) coating the hair with a composition comprising (A) an organosiloxane polymer having an organic substituent containing a nitrogen-hydrogen bond, which polymer is emulsified in water with a surfactant and (B) a water solubilized additive selected from the group consisting of organic titanates, zirconates, germanates and mixtures thereof;

(II) imposing a desired configuration on the hair; and

(III) drying the coated hair in the desired configuration.

European Patent Office

# EUROPEAN SEARCH REPORT

0117360

Application number

EP 83307752.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP - A1 - 0 055 606 (TORAY SILICONE COMPANY LIMITED)<br>* Totality, especially claims 1-4, page 6, last paragraph * | 1-4 | C 08 L 83/08<br>A 61 K 7/11<br>A 61 K 7/06 |
| Y | * Claim 1 *<br>-- | 5 | |
| Y,D | GB - A - 2 058 103 (BRISTOL-MYERS COMPANY)<br>* Abstract; examples; claims 1,3 *<br>-- | 5 | |
| A | US - A - 4 344 763 (E. TOLGYESI et al.)<br>* Abstract; examples *<br>---- | 1,5 | **TECHNICAL FIELDS SEARCHED (Int. Cl. 3)**<br>C 08 L 83/00<br>A 61 K 7/00 |

The present search report has been drawn up for all claims

| Place of search | Date of completion of the search | Examiner |
|---|---|---|
| VIENNA | 27-04-1984 | KALTENEGGER |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document